(19) Europäisches Patentamt
European Patent Office
Office européen des brevets

(11) **EP 3 838 112 A1**

(12) **EUROPEAN PATENT APPLICATION**

(43) Date of publication:
**23.06.2021 Bulletin 2021/25**

(51) Int Cl.:
***A61B 3/00*** *(2006.01)*

(21) Application number: **19217946.3**

(22) Date of filing: **19.12.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority: **18.12.2019 HU 1900433**

(71) Applicant: **Medicontur Kft.**
**2072 Zsámbék (HU)**

(72) Inventors:
• **Ábrahám, György István**
  **1118 Budapest (HU)**
• **Fekete, Róbert Tamás**
  **1214 Budapest (HU)**
• **Kontur, László**
  **1142 Budapest (HU)**
• **Koncsár, Péter**
  **1119 Budapest (HU)**

(74) Representative: **Kacsuk, Zsófia**
**Kacsukpatent Kft.**
**Üteg u. 11/a**
**1139 Budapest (HU)**

(54) **METHOD OF CALIBRATING A COMPUTERISED COLOUR VISION TEST AND METHOD OF TESTING COLOUR VISION ON A COMPUTER**

(57)    The invention relates to a method of calibrating a colour vision test for testing colour vision under given ambient lighting conditions, which colour vision test is to be displayed on a colour screen (12) of a computer (10) having at least one input interface (14), characterised by displaying a calibration test on the screen (12) under given ambient lighting conditions to a person with normal colour vision before starting the colour vision test,

displaying within at least one measuring region (31) of the screen (12) a colour determination task requiring user input as a part of the calibration test, reading the user input through at least one input interface (14) of the computer (10),

evaluating the read user input and determining as a result of the evaluation a display error resulting from a combination of a colour reproduction capability of the screen (12) and an effect of the ambient lighting conditions on colour vision, and

determining a modification to the colour vision test from the display error that corrects the colour vision test with respect to the display error.

The invention further relates to a method of testing colour vision using the calibration method, as well as to a computer and computer program configured for performing such methods.

S10 — Displaying calibration test

S101 — Instructions to adapt eye

S102 — Colour determination exercise

S11 — Reading user input

S12 — Evaluating user input

S13 — Display error

S14 — Determining modification of colour vision test

Fig. 3

EP 3 838 112 A1

**Description**

The invention relates to a method of calibrating a colour vision test for testing the colour vision of a person with colour vision deficiency, which colour vision test is intended to be displayed on a colour screen of a computer having at least one input interface.

The invention also relates to a method of testing colour vision using the calibration method, as well as to a computer program configured for performing such methods and devices storing this program.

In the context of the present invention computer is understood to mean an electronic device comprising hardware and software components that is for receiving, storing, processing and transmitting data, and program code containing appropriate computer commands for performing these tasks. The computer typically has at least one processor, memory, and peripherals, i.e. input and output interfaces, the latter of which may be provided in an integrated way, such as in the form of a touchscreen. In the context of the present invention a computer is particularly a desktop PC (Personal Computer), laptop, tablet, smartphone, etc.

Colour vision is ensured by certain receptors located on the retina, namely the cones providing daylight vision. There are three types of such receptors based on their spectral sensitivity. L-cones are sensitive mainly to the long wave (red) range of the spectrum. M-cones are sensitive to light in the medium wavelength range (green), while S-cones are sensitive to light in the shortwave range (blue). The sense of colour is created on the basis of the relative values of the stimuli transmitted by the colour-sensing L, M and S-cone receptors.

Colour vision deficiency (also called colour blindness) is caused by the spectral sensitivity curves of colour blind persons deviating to smaller and greater extents from those of normal (healthy) people, accordingly the most frequent forms of colour vision deficiency are protanomaly, protanopia, deuteranomaly and deuteranopia. The colour-sensing problem protanomaly is caused by the spectral sensitivity of the L-cones being closer to the spectral sensitivity of the M-cones than in the case of those with normal colour vision. As a consequence, the difference between the stimuli of the L and M-cones is reduced, and this results in a deterioration of the ability to differentiate between colours. In extreme cases the spectral sensitivity of the L-cones is shifted to such an extent that it coincides with the spectral sensitivity curve of the M-cones, which is referred to as protanopia.

In the case of deuteranomaly the spectral sensitivity of the M-cones lies closer to the spectral sensitivity of the L-cones, than in the case of those with normal colour vision. The result is similar to the previous case: the difference between the stimuli of the L-cones and M-cones drops, i.e. the ability to differentiate between colours deteriorates in this case too. Deuteranopia is the condition when the spectral sensitivity curve of the M-cones is shifted to such an extent that it coincides with the spectral sensitivity curve of the L-cones.

Approximately 23% of people suffering from colour vision deficiency have protanomaly, 73% have deuteranomaly, and about 4% have severe colour blindness (protanopes and deuteranopes). The most severe form of colour vision deficiency monochromasia, total colour blindness is extremely rare. The blue sensitive receptor, the S-cone is very rarely defective. Damage to this receptor is usually caused by illness or intoxication, and when the cause is eliminated colour vision becomes normal once again. Due to this almost 100% of people with defective colour vision have protanomaly, deuteranomoly, protanopia or deuteranopia.

Computers are being used increasingly frequently to test colour vision. Patients are asked to perform various colour tests on these computers to determine if they have any colour vision deficiency and, if so, its type and severity. Such tests include the screen versions of the well-known pseudochromatic test books (e.g. Ishihara, Rabkin, Dvorin, Velhagen, Hardy, HRR) and of various colour identification tests. Even though electronic screens display colours that differ from screen type to screen type and which are different to those in printed or painted tests, their use is still spreading, partly because of their increasingly better colour reproduction abilities and partly because of their easy access. A further advantage is that electronic display devices make it possible for persons located at remote places around the world and potentially having impaired colour vision, to be able to diagnose themselves (or have telediagnosis performed), on the basis of which a diagnosis may be determined and any suitable correction device (colour filter glasses) may be ordered on the internet.

However, the different colour reproduction capabilities of electronic displays represent a great problem, as they have different primary colours depending on the manufacturer, both with respect to the characteristic wavelength and the spectra of the primary colours.

Another problem is the degree of adaptation of the tested person's eye to the ambient light. It is not indifferent whether the sensitivity of each of the three receptors (red, green, blue) of a human eye is the same, or significantly different from each other as a result of different ambient lighting conditions.

The same eye sees colours in different ways in different states of adaptation. For example, the human eye sees different colour shades in incandescent light, sunlight, fluorescent light, and in light emitted by LEDs. Due to this if a colour vision test program is displayed on a computer monitor, laptop, tablet or smartphone, then depending on the device's colour reproduction capability and the degree of adaptation of the tested person's eye to the ambient lighting conditions the test may produce incorrect results and therefore an incorrect diagnosis.

It is an object of the present invention to provide a method and computer program to overcome the problems associated with the prior art, in particular, the problems described above.

The invention is based on the recognition that a person with normal colour vision can contribute to making a correct diagnosis of a person with colour deficiency who is present at the same location as the colour blind person being tested and who uses the same device under the same ambient lighting conditions, whereby the measured results provide information both about the device and about the ambient lighting conditions. The same test may be performed on the reference person with normal colour vision as is then performed on the patient to be measured, or a different test may be separately performed for the purpose of calibration.

The colour vision test may be modified in view of the calibration measurement results of the person with normal colour vision in order to take into consideration the screen's colour reproduction capability and the effect of the ambient lighting on colour vision. Two things may be done with the measurement results of the person with normal colour vision: the results, such as the monitor's R/G/B settings, may be used when performing a given task for the ad hoc modification and tuning of the parameters of the software colour vision test performed on the patient, in this way the test for examining colour vision is now displayed to the patient with modified parameters. The other possibility is that the displaying of the colour vision test is not modified, however a different diagnosis is made from its results after performing the necessary correction.

The above objectives are achieved in accordance with the invention by the calibration method according to claim 1.

The invention further relates to a colour vision testing method according to claim 9 which uses the calibration method according to the invention.

The invention further relates to a calibration method, and optionally the computer according to claim 12 configured to perform the measuring method, and the computer program according to claim 14.

Advantageous embodiments of the invention are defined in the attached dependent claims.

Further details of the invention will be explained by way of exemplary embodiments with reference to the Figs. , wherein:

> Fig. 1 depicts a schematic view of exemplary computers for implementing the method according to the invention,
> Fig. 2a depicts a schematic view of the subpixels of an exemplary screen,
> Fig. 2b depicts a schematic view of the subpixels of a different exemplary screen,
> Fig. 2c depicts a schematic view of the subpixels of a third exemplary screen,
> Fig. 3 depicts a flow diagram of an embodiment of the calibration method according to the invention,
> Fig. 3a depicts a flow diagram presenting the supplementary steps of an exemplary embodiment of the calibration method according to the invention,

> Fig. 4 depicts a schematic screen image of an exemplary embodiment of a calibration test according to the invention,

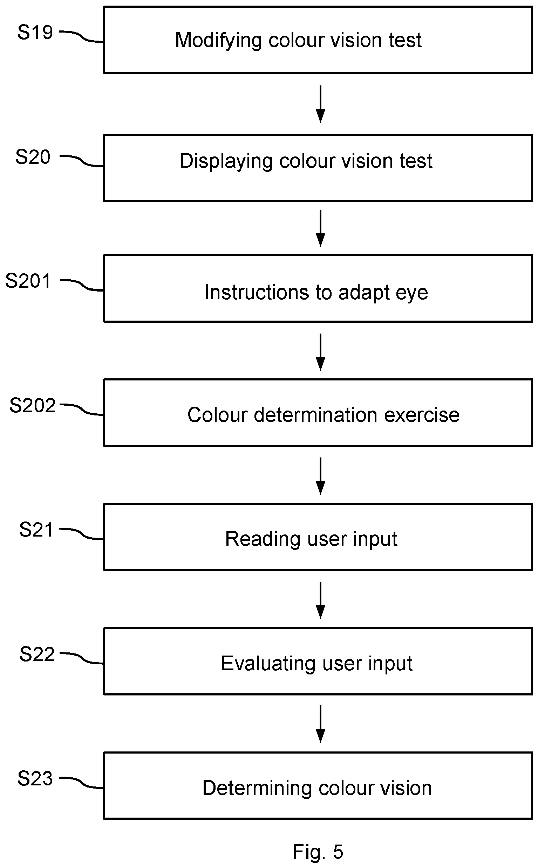

> Fig. 5 depicts the flow diagram of an exemplary embodiment of a colour vision test according to the invention,
> Fig. 6 depicts a schematic screen image of an exemplary embodiment of a colour vision test according to the invention,
> Fig. 7 depicts a schematic screen image of a different exemplary embodiment of a colour vision test according to the invention,
> Fig. 7a depicts an illustrative guide for correcting the displaying of the colour vision test according to Fig. 7.

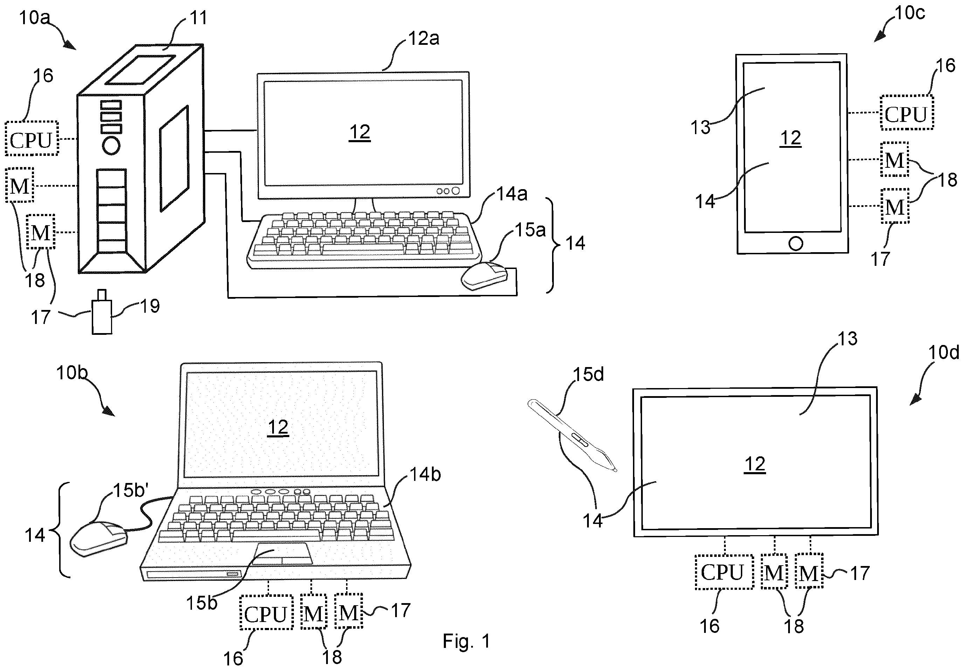

Fig. 1 shows a schematic view of exemplary computers 10 for implementing the method according to the invention. Reference sign 10a designates a desktop PC, which is provided with peripherals in the conventional way, such as a monitor 12a with a screen 12 and input interfaces 14, in the present case keyboard 14a and mouse 15a. Naturally the PC 10a may also have other input interfaces 14 and peripherals.

Reference sign 10b designates a laptop, which has a built in screen 12, built in keyboard 14b and built in mouse 15b, as input interfaces 14, in addition the exemplary laptop 10b shown in Fig. 1 also has an external mouse 15b'. Naturally, the laptop 10b may also have other input interfaces 14 and output interfaces.

Reference sign 10c designates a, which has a built in screen 12 in the form of a touchscreen 13 operating as input interface 14 and output interface at the same time. In addition other peripherals may be connected to the smartphone 10c.

Reference sign 10d designates a tablet, which also has a built in screen 12 formed as a touchscreen 13, therefore this also operates as both an input interface 14 and output interface at the same time. In addition the tablet 10d ha a further input interface 14 in the form of a touch pen 15d, which enables more precise use of the touchscreen. Naturally further peripherals are also possible here.

At least one processor 16 and storage media 17 are located inside the computers 10 which have been marked schematically with dashed lines in Fig. 1 in the present case the storage media 17are memories 18. In the case of the desktop PC 10a the processor 16 and the memories 18 are arranged in a separate housing 11. It is usual to call the central processor 16 a central processing unit (CPU), the text "CPU" in the text box delimited with dashed lines schematically illustrating the processor 16 makes reference to this. However, the at least one processor 16 may be provided in any known form, for example as a part of a system on chip, which may also have integrated memories 18. This latter component is customarily abbreviated as SoC (system on chip). There may be several processors within one computer 10, an example of this being a so-called multicore processor, which is actually one chip within which the individual cores are also processors 16 themselves.

From the point of view of retaining the data the memories 18 may be of two types: volatile and non-volatile. Non-volatile memory 18 is a storage medium 17 (background storage) that is adapted for permanently storing program codes (programs in short) containing computer commands that may be executed (run) by the processor 16. The very fast access memory 18 actively used by the processor 16 during the running of programs is usually volatile. While the program is being run or as a result of this the computer 10 receives data (for example it reads data via the input interface 14), stores data (temporarily in the volatile memory 18, or long-term in the background storage), processes data and transmits it (such as to an output interface, for example it displays data on the screen 12).

Further customary elements may also be in the computer 10 in the way known by a person skilled in the art, such as data buses, switches, connections, etc., which are not separately shown here.

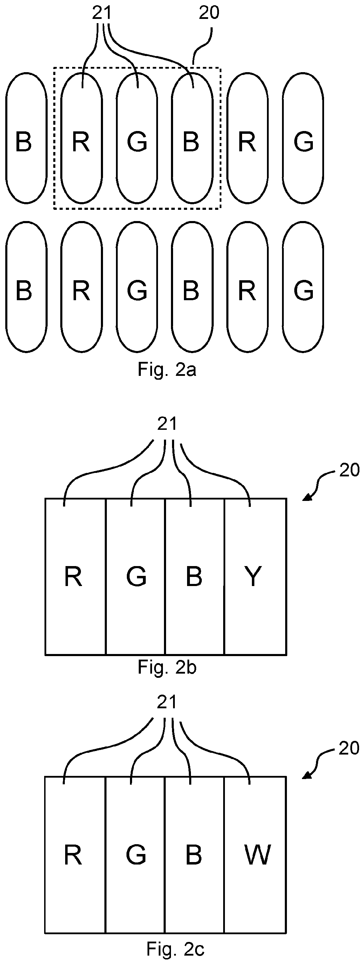

The screen 12 of the computer 10 used for the implementation of the present invention is a colour screen 12, which means that each of the pixels 20 forming the image dots of the screen 12 are made up of several, differently coloured so-called subpixels 21, as illustrated in the enlarged detail of an exemplary screen 12 in Fig. 2a, in which the neighbouring subpixels 21 forming one pixel 20 have been framed with a dashed line. The pixels 20 of screens 12 typically contain subpixels 21 in three colours: red, green and blue subpixels 21, which are marked respectively by the letters R, G and B in Fig. 2a. The technology used to display the individual colours depends on the type of screen 12, this may take place, for example, using appropriately coloured organic light-emitting diodes (OLED), as in the case of OLED screens 12, or, for example, using controllable liquid crystal filters and white background illumination, as in the case of LCD type screens 12. It should be noted that the use of other screen technologies is conceivable, such as QLED or plasma technologies, as is obvious for a person skilled in the art. There are also LCD screens in existence that in addition to the conventional red, green and blue subpixels 21 also contain other coloured subpixels, for example in the Quattron brand LCD screen there is a fourth, yellow coloured subpixel 21 within each of its pixels 20. This is marked with the letter Y in Fig. 2b. In the case of the LCD screens illustrated in Fig. 2c using the so-called PenTile RGBW technology, the fourth subpixel 21 marked with W is controllable to become transparent, in this way it transmits the white background light without it passing through a colour filter, in other words this type of screen 12 uses white as the fourth colour.

The calibration test and the colour vision test according to the invention are carried out by a computer program on the computer 10 used by a user. The two types of test may be implemented with separate computer programs, or within the framework of a single computer program. The computer program(s) may be stored in the non-volatile memory 18 of the computer 10 or they may be read from a separate non-volatile storage medium 17, such as a USB flash drive 19 shown in Fig. 1, which has been indicated beside the desktop computer 10a. The external storage medium 17 may, alternatively, be a CD, DVD, Memory Stick, etc. An embodiment is also conceivable in which the computer program(s) are stored on a remote computer, typically a server, and, for example, the displaying of the given test is realised on the screen 12 of the local computer 10 through an Internet connection, and the transmitting of the user input data read through the local input interfaces 14 to the program(s) running on the server for processing also takes place via the Internet.

It is clear for a person skilled in the art that computers that are also adapted for implementing the method according to the invention but that are different to the computers 10 presented here are also conceivable.

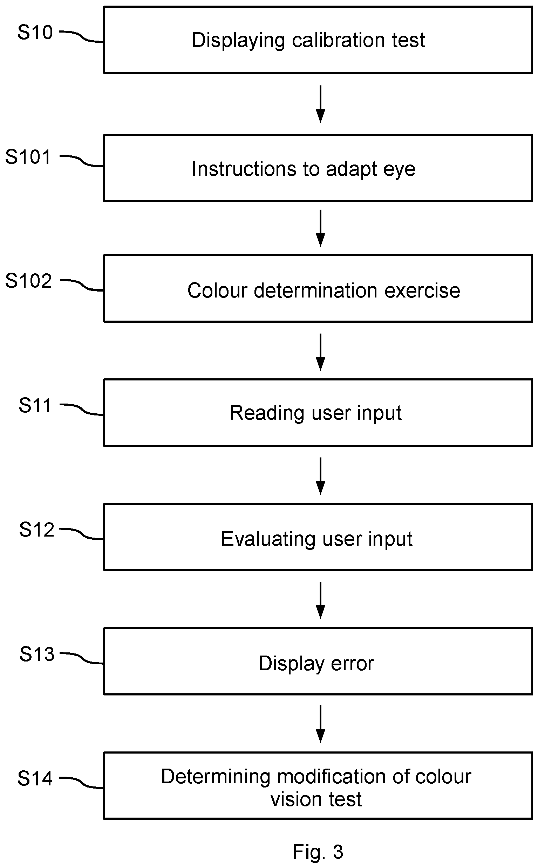

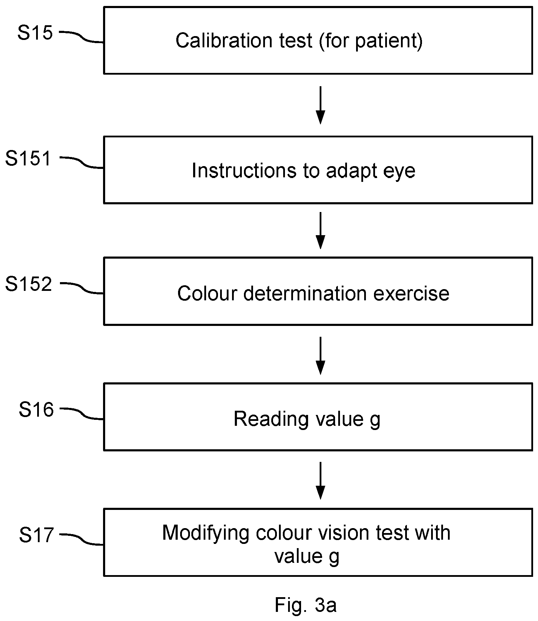

An exemplary embodiment of the method according to the invention is presented using the flow diagram shown in Fig. 3.

During the method a computer 10 is used that has a colour screen 12 and at least one input interface 14. A colour vision test for testing the colour vision of a colour blind person is to be displayed on the screen 12 of the computer 10 under given ambient lighting conditions, however calibration is required before this takes place in order to be able to take into consideration the effect on colour vision of the colour reproduction capability of the given screen 12 and the effect of the given ambient lighting conditions on colour vision, which both influence the colour vision test as explained previously. The given ambient lighting may be natural light (e.g. daylight within a room) or artificial light (e.g. a room illuminated with the light of a lamp). The calibration relates to the performance of the colour vision test under given ambient lighting conditions, therefore it is required that the given ambient light conditions do not change between the time when the calibration test is displayed and when the colour vision test is displayed later. For this in step S10, before the colour vision test is started, a calibration test is displayed on the screen 12 in the same ambient lighting conditions for a person with normal colour vision.

Anyone may be invited to perform the calibration test in the environment of the patient to be tested who knows they are not colour blind, i.e. anyone who has normal colour vision. If no one in the environment of the patient to be tested knows this for sure about themselves, then it is preferable to invite two female persons to perform the calibration test, as the proportion of females with colour vision deficiency is significantly lower (approx. 0.4%) than in the case of males, therefore the probability that both of the females have colour vision deficiency, and in the same way, is negligible. Apart from this very rare situation, if for example, one person invited to do the calibration test has colour vision deficiency, then from the different results of the two calibration tests it can be determined that at least one of the person has colour vision deficiency, hence the calibration will be unsuccessful and another person or pair of persons will have to be found for the calibration. All this, however, does not make the method according to the present invention unusable, all it means is that the method will be unsuccessful in a very small percentage of cases (if, for example, the results of the calibration tests performed on the two persons are contradictory) or it will lead to an incorrect result (if, for example, persons performing the calibration test who think they have normal colour vision but in fact they have colour vision deficiency). The person with normal colour vision and their selection are not a part of the method according to the invention, in other words step S10 of the method according to the present invention only extends to displaying the calibration test on the screen 12, which is designed for a person with normal colour vision, and which, therefore should be performed by a person with normal colour vision. In the following the person participating in the calibration test will be referred to as the user.

During the displaying of the calibration test in step S10, in step S101 an instruction is given to the user (in other words the person with normal colour vision) which when performed ensures that his or her eyes have become adapted to the given ambient lighting conditions. For example, a text instruction is displayed on the screen for the user of the calibration test telling the user to look at an object 40 known to him or her to be white, such as a sheet of white paper, for a few minutes, preferably for at least 2 minutes and optimally for 10 minutes. Colour vision is partially a brain signal processing task, therefore in spite of the possible yellowish, bluish, etc. toned light of the ambient illumination, the human brain is able to adjust the white balance, which in practice means that the sensitivity ratios of the S, M and L-cone receptors are tuned so that after a certain time the user sees the colour of an object 40 known to be white as white even though the individual wavelength components measured by an instrument of the light reflected from the object 40 are present in differing intensities, in other words the light reflecting from the object 40 has an objective coloured tone (for example light yellow in a yellowish lamp light).

The instruction for the user may not only be displayed during the calibration test and not only on the screen 12. For example, it is conceivable that it has been provided previously as part of the instructions for use, either in electronic or paper form.

It is not possible to guarantee the adaptation of the eyes of the user performing the calibration test to the ambient lighting conditions, but, for example, the probability that the users eyes have appropriately adapted can be increased with the above instruction, and so the calibration will be more precise. The eyes will still have a degree of adaptation if the user is not asked to perform an exercise that adapts his or her eyes to the ambient lighting conditions, hence the calibration can still be performed, and can be used for ameliorating the colour vision test, but calibration may be less precise from the point of view of taking into account the effect of the ambient lighting conditions on colour vision.

The user is also preferably instructed to maintain and refresh the adaptation of the eyes while performing the calibration test, for example, by returning to look at the white colour (the object 40) for a longer period of time than the amount of time spent looking at the screen 12 to prevent the eyes from becoming "disadapted" due to looking at the colours on the screen 12 for a long time during the test. If a white sheet of paper or other known white object 40 is not used, instead the automatic adaptation of the eyes to the environment is relied on, it is still preferable to instruct the user to look away from the screen 12 and to the environment from time to time while performing the calibration test. Naturally, it is more effective if the user looks at an object 40 he or she knows to be white, because this way adaptation is faster.

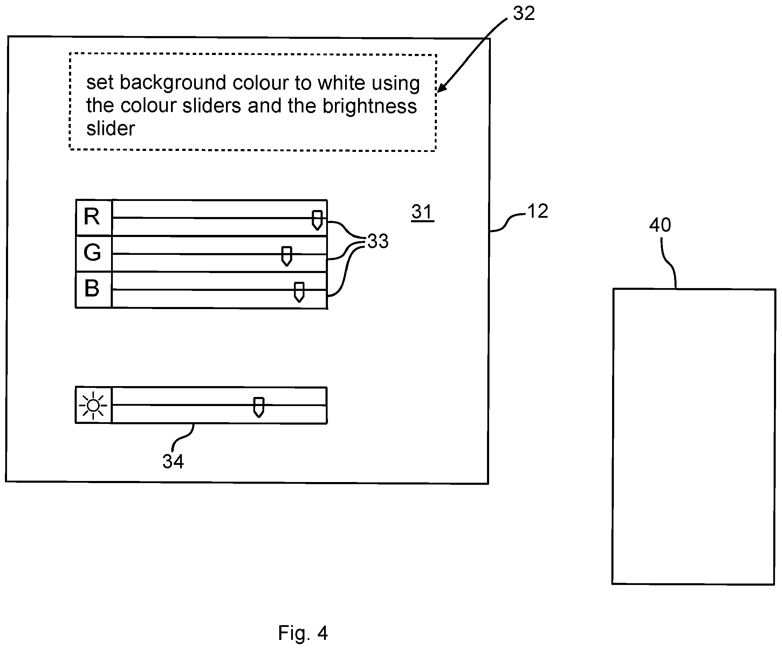

During the displaying of the calibration test in step S10, in step S102 a colour determination task is displayed requiring user input in at least one region of the screen 12. Henceforward this region will be referred to as "measuring" region. Fig. 4 presents the screen image of an exemplary colour determination task displayed on the screen 12. This may be, for example, the screen image of the monitor 12a of the desktop PC 10a. Optional text instructions 32 for the user (marked with a dashed line frame) are displayed on the screen image, preferably in front of the entire background of the measuring region 31, in other words the background of the text instructions 32 is also a part of the measuring region 31. The instructions 32 may also contain instructions relating to eye adaptation, or only the instructions related to the execution of the calibration test.

In the case of this example there are sliders 33 displayed for adjusting the intensity of the red, green and blue subpixels 21. If the screen 12 has further coloured subpixels in addition to the red, green and blue subpixels 12, then their intensity is preferably set to 0 for the purposes of performing the colour determination task, and the adjustment of these additional coloured subpixels is not made available to the user. The user is able to change the positions of the sliders 33 via one of the input interfaces 14 of the computer 10 (e.g. the mouse 15a, 15b, 15b' or by using a finger or the touch pen 15d on the touchscreen 13). The colour of the measuring region 31 is adjusted by regulating the intensity of the subpixels 21 in the measuring region 31, preferably according to the actual intensities determined by the sliders 33, therefore the user sees the colour mixed by him or her in the measuring region 31 in real time. Therefore, in this case the user input is the adjustment of the sliders 33, which the user provides as a response to the colour determination task, and which in step S11 is read via one of the input interfaces 14 of the computer 10.

During the performance of an exemplary colour determination task in step 101 the user must set the colour of a white object 44 observed for a prolonged period of time within the measuring region 31 by adjusting the sliders 33, in other words by adjusting the intensity of the red, green and blue subpixels 21. The text instructions 32 displayed on the screen 12 may relate to this. Preferably, while setting the colour of the white object 40 the user may also be instructed to take care to look at the object 40 for longer periods of time and at the screen 12 for shorter periods of time, so that the illumination of the screen 12 has the least possible effect on the degree of adaptation of the eyes. If the user looks at the screen 12 for a prolonged time, the degree of adaptation of the eyes to the ambient light will be reduced, and the calibration will be less precise from the point of view of taking into consideration the effect of the ambient lighting conditions.

When setting the white colour of the measuring region 31 it may happen that although the colour of the measuring region 31 is now similar to the colour of the reference white object 40, it is still lighter or darker than that. In order to correct this a further slider 34 is preferably provided with which the brightness of the measuring region 31 may be adjusted. Optionally it may also be made possible to adjust the hue of the screen 12 and its saturation as well.

The user input read in step S11 is evaluated in step S12, and as a result of the evaluation the display error resulting from the combination of the colour reproduction capability of the screen 12 and the effect of the ambient lighting conditions on colour vision is determined in step S13. It is not intended to separate the effect of the colour reproduction capability of the screen 12 on colour vision and the effect of the ambient lighting conditions on colour vision from each other or to determine them separately, as these jointly influence how the user sees the colours appearing on the screen 12.

During the evaluation account is preferably taken of the fact that the majority of problems related to colour vision are manifested in mistaking the colours red and green, therefore in the case of the above example the evaluation preferably consists of determining the r and g values of the intensity of the red and green subpixels 21 from the positions of the sliders 33, and optionally of the slider 34, set by the user, which in standard depiction are each a number between 0 and 255, then the difference of these is taken. Furthermore, account may also be taken of the read brightness value br, which refers to how bright the pixels of the screen 12 are. In step S13 the result of the evaluation will be the r-g value, which is regarded as the display error resulting from the combination of the colour reproduction capability of the screen 12 and the effect of the ambient lighting conditions on colour vision.

Naturally, the colour vision test may be aimed at measuring the very rare colour vision deficiency linked to errors of the S-cone, in this case during calibration the b value relating to the intensity of the blue subpixels 21 is taken into account, and from this, for example, the r-b, or b-g value as display error is determined as well.

Optionally the result of the evaluation may also contain the r-g, r-b and b-g values, as the various components of the display error.

In step S14 a modification to the colour vision test is determined from the display error that corrects the colour vision test with respect to the display error.

For example, the result of the calibration test according to the above example is to be used for a colour vision test for measuring red-green colour vision deficiency, in the case of which the patient being tested needs to set the precise colour yellow (i.e. a yellow with no trace of orange or green) by using the sliders 33 regulating the intensity of the red and green subpixels 21 while the intensity of the blue subpixels 21 is reduced to 0 with the slider 33 that regulates the intensity of the blue subpixels 21.

Mainly two things may be determined as the modification to the colour vision test. On the one hand it is possible to determine a modification to the parameters of the colour vision test so as to correct the parameters relating to the intensity of those coloured subpixels 21 that were taken into account in the display error when the colour vision test is displayed on the screen 12. In other words, in the case of this example, when the colour vision test is displayed the r-g difference calculated during calibration is added to the value of the intensity of the red subpixels 21 in the interest of correcting the display error, therefore while setting the yellow colour the corrected yellow result is displayed on the screen 12 to the tested patient. In this way it is possible to read as the user input the position to which the patient sets the slider 33, and from this a correct diagnosis may be established, because it is in fact the patient's colour vision deficiency that is being measured and not the display error.

On the other hand it is also possible to not modify the displaying of the colour vision test, instead as an modification to the colour vision test a modification to the evaluation of the colour vision test is determined that corrects the measurement result obtained with the colour vision test with respect to the display error in terms of the intensity of the coloured subpixels 21 taken into account in the display error during calibration when evaluating the colour vision test. In the case of the present example this means that the r-g difference obtained during calibration is subsequently subtracted from the result belonging to the yellow colour set by the tested patient (i.e. subtracted from an $r_2$ value set for the intensity of the red subpixels 21). As the "result" is a value close to zero (precisely zero in the case of a person with normal vision and an ideal screen 12), therefore by subtracting the display error from this result as the correction, the correct diagnosis of the person with colour vision deficiency is established.

A colour vision test is also a part of a preferred embodiment of the invention. In this case the calibration method is executed before the colour vision test is started under given ambient lighting conditions, then in step S19 the modification determined during the calibration method is carried out, which in accordance with the above may involve the modification of the display parameters of the colour vision test or the modification of the evaluation parameters.

In step S20 the modified colour vision test is displayed on the screen 12. In the present example the colour vision test is very similar to the calibration test, therefore, instead of making unnecessary repetitions reference will be made to the details of the calibration test, and if the colour vision test is not in contradiction with it, the description of the calibration test is valid here also.

The result of the calibration may be improved if care is taken to ensure that the tested patient's eyes are also adapted to the same ambient lighting conditions before he or she performs the colour vision test displayed on the screen 12. For this, in step S201 during the colour vision test (or optionally before it) instructions are preferably given to ensure the patient's eyes are adapted to the ambient lighting conditions, for example the patient is instructed to look at an object that he or she knows to be white for a few minutes, preferably for at least 2 minutes, optimally for 10 minutes, preferably the same white object 40 that the person with normal colour vision used for the calibration test. Similarly to the calibration method, the instructions ensuring adaptation of the eyes may be given to the patient in another form as well.

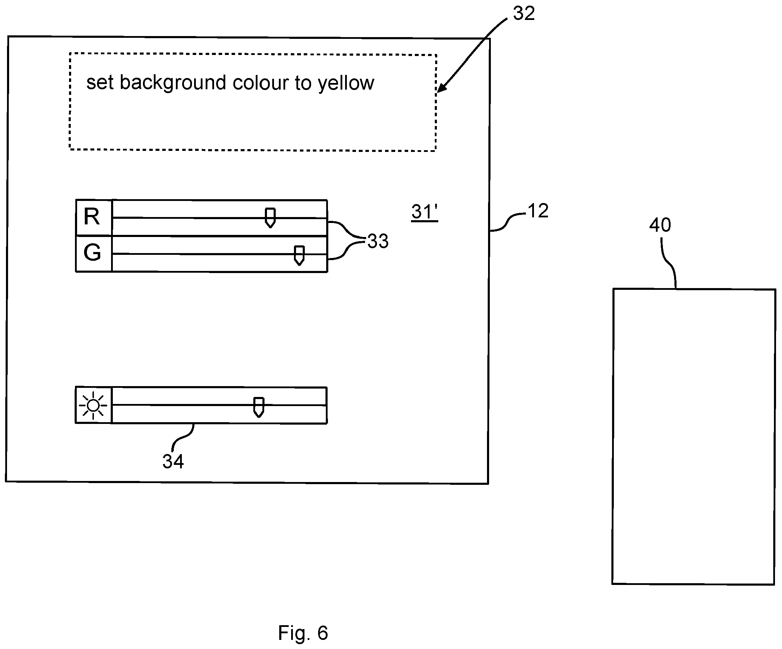

In the case of this example in step S20 during the displaying of the colour vision test in step S202 a colour determination task requiring user input for measuring red-green colour vision deficiency is displayed on the screen 12 the schematic screen image of which is presented in Fig. 6. In the case of this task the patient needs to set the colour yellow (i.e. a yellow with no trace of orange or green) in the measuring region 31 of the screen 12 by using the sliders 33 regulating the intensity of the red and green subpixels 21. An instruction 32 corresponding to this is displayed on the screen (marked with a dashed line frame). During performance of the task the intensity of the blue subpixels 21 is reduced to 0, and in the case of the present embodiment there is no slider 33 with which the user could adjust the colour blue. The intensity of any further coloured subpixels is also set to 0, and it is not made possible for the user to change this. Preferably in this case a separate slider 34 is provided for adjusting brightness.

In this case the user input is the adjustment of the sliders 33 and 34, which in step S21 is read via one of the computer's 10 input interfaces 14.

The user input read in step S21 is evaluated in step S22, and as a result of the evaluation the patient's colour vision is determined in step S23. The latter also includes the type and severity of the colour vision deficiency.

In the case of the presented example the calibration test was different from the colour vision test, however an example is conceivable in the case of which the calibration test is the same as the colour vision test. For example, during the calibration test the task of the person with normal colour vision acting as user is also to mix the colour yellow (preferably after resting his or her eyes on the white reference object 40).

A further possibility illustrated in Fig. 3a is that the tested patient is also asked to perform the calibration test, in this way the colour vision test may be simplified. For example, as the completion of the calibration in step S15 the calibration test is also displayed for the tested patient, during which in step S151 the patient is instructed to look at the white reference object 40 that the person with normal colour vision also used for some minutes, optimally for approximately 10 minutes, then in step S152 the patient is also instructed to perform the white setting task described above, and in step S16 the user input relating to the setting of the slider 33 regulating the intensity of the green colour is read, in other words the g value set by the patient, is read. With this g value the colour vision test relating to the setting of the colour yellow is modified in step S17 in such a way that the value of the green subpixels 21 in the measuring region of the displayed colour vision test is set to g, and compared to this the patient has to set the intensity of the red subpixels 21 in such a way as to mix the colour yellow. By fixing the intensity of the colour green it is possible to use approximately the same control range of the screen 12 which has non-linear characteristics.

Other types of colour determination tasks may be displayed both during the calibration test and the colour vision test. In the case of the above examples, the task involved either the person with normal colour vision or the tested patient setting a given colour in the measuring region 31 of the screen 12 by adjusting the intensities of the various coloured subpixels 21. However, there may be many types of colour determination tasks, the tasks may involve, for example, adjusting the intensity of more or fewer coloured subpixels 21, for example, the user only has to adjust one type of coloured subpixel 21, while the intensities of the other coloured subpixels 21 are set to a specific value for the user. The colour determination task may involve, for example, selecting one or more colour samples from a set of colour samples displayed in the measuring region 31 according to specific instructions (for example, selecting the colour sample that the user sees as being more orange from among two different shades of yellow colour).

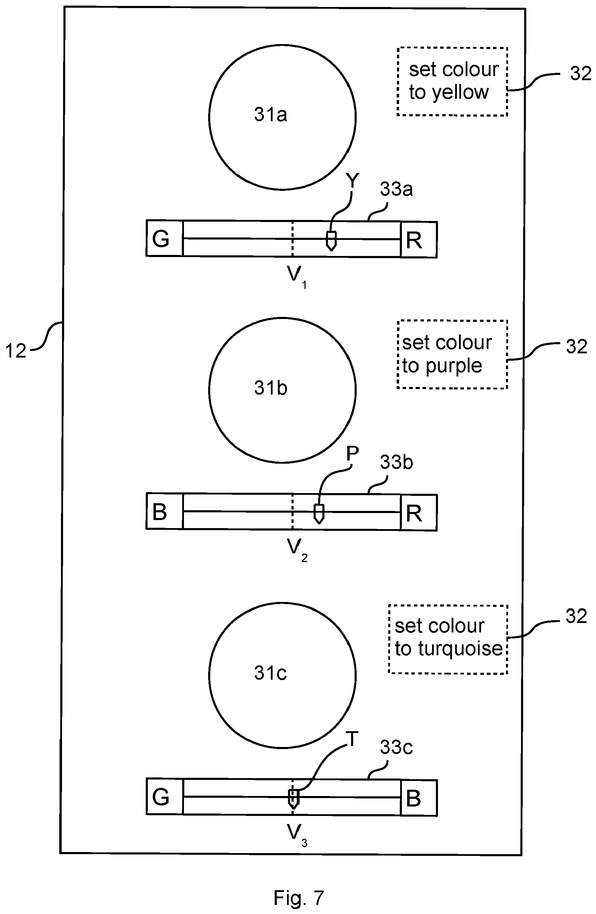

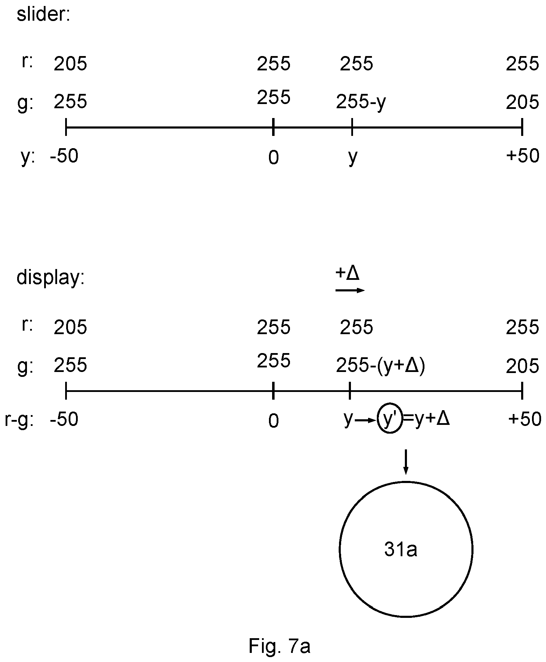

Fig. 7 shows an illustration of the screen image of a more complex colour determination task as it appears on the screen 12 of a smartphone 10c used as the computer 10. In the case of this example the measurement region 31 consists of three circular surfaces 31a, 31b, 31c, the colour of which may be changed using the slider 33a, 33b, 33c displayed under each of them. The colour yellow needs to be set in the first circular surface 31a by changing the position Y of the first slider 33a. The central position of the slider 33a indicated with a dashed line V1 would result in the colour yellow in the case of an ideal screen 12, in this case the intensity of the red subpixels 21 and of the green subpixels 21 within the circular surface 31a are both 255 (on the standard scale of 0-255). The intensity of the blue subpixels 21 is reduced down to 0 within the circular surface 31a at any position of the slider 33a. By moving the slider 33a to the right (towards the terminal position marked by R) as compared to the central position, the intensity of the red subpixels 21 does not change, while the intensity of the green subpixels 21 drops evenly down to 205 within the circular surface 31a, therefore in this case an increasingly orange yellow will appear as the proportion of the intensity of the colour red will be increasingly greater as compared to that of the colour green. By moving the slider 33a to the left (towards the terminal position marked by G) the intensity of the red subpixels 21 drops evenly down to 205, while the intensity of the green subpixels 21 remains constant within the circular surface 31a, therefore in this case an increasingly green shade of yellow will appear as the proportion of the intensity of the colour green becomes increasingly greater as compared to that of the colour red.

The current position Y on the slider 33a is described with a value between ± 50, in such a way that the central position marked with a dashed line is viewed as 0, in the right terminal position the intensity of the a red subpixels 21 is 50 more than the intensity of the green subpixels 21, therefore this is viewed as the value +50, while in the left terminal position the intensity of the red subpixels 21 is 50 less than the intensity of the green subpixels 21, and this is viewed as the value -50.

Naturally, sliders 33a could be used on which the intensity of the red and green pixels can be adjusted over the entire scale of 0 to 255, however in the case of a colour vision test displayed on a small sized screen 12 (such as the screen 12 of a smartphone 10c) this would result in a small movement of the slider 33a causing a significant colour tone change within the circular surface 31a, in other words the patient would not necessarily be able to precisely mix the colour he or she sees as yellow. In order to overcome this problem it is preferred to allow moving of the slider 33 only in the expected range around yellow, this is why in the case of this example the scale between ± 50 presented above was chosen. Naturally a scale wider than this may be chosen, for example, moves between the values ±100.

If during the calibration method the adjustment of brightness has also been made possible on the slider 34, then this result may be used to provide more precise measurement in such a way that, for example, the 255 - 255 value of the red and green colours are reduced in accordance with the value of brightness. For example, 95% was set for brightness by the person with normal colour vision in the calibration test using the slider 34, then correspondingly the 255 - 255 value is reduced to the value 243 - 243 (which represents the correction of one of the components of the display error). In this case in the right side terminal position the value of the intensity of the green subpixels 21 will be 193, and in the left side terminal position the value of the intensity of the red subpixels 21 will be 193. Naturally many forms of scaling are conceivable, henceforward, for the sake of simpler illustration, the original example will be used, in other words at the central position marked with the dashed line V1 the intensity of both the red and the green subpixels is 255 within the circular surface 31a, while the intensity of the blue subpixels 21 is 0. If the brightness value set during calibration is also to be taken into account, then according to that described above the intensity corresponding to 255 will be proportionately reduced, and in the examples to be explained in the following this reduced value will be used instead of the maximum value of 255.

Similarly, in the second circular surface 31b the task is to set the colour purple using the second slider 33b, which when in its central position marked with the dashed line V2 the intensity of the blue subpixels 21 and of the red subpixels 21 within the circular surface 31b is 255, while the intensity of the green subpixels 21 in any position of the slider 33b is 0. The slider 33b being in the terminal position marked by B results in a bluish shade of purple, and the slider being in the terminal position marked by R results in a reddish shade of purple in such a way that, analogous to that described above, the scale here runs between the values ±50. The set position on the second slider 33 is marked with P.

In the third circular surface 31c the task is to set the colour turquoise, which in an ideal case is created at the central position marked with the dashed line V3, where the value of both the blue and the red subpixels 21 is set to 225 within the circular surface 31c. In an analogous way to that described above the third slider 33c being in the terminal position marked by B results in a bluish shade of turquoise, while the terminal position marked by G would result in a greenish shade of turquoise within the circular surface 31c, and a value within the scale of± 50 corresponds to an arbitrary position T.

The values belonging to the Y, P and T positions set by the user are referred to with the letters y, p and t, respectively.

In an ideal case a person with normal vision would set the value of 0 on all three sliders, while in the case of persons with protan or deuteran colour vision deficiency the y, p and t values would be set as follows:

Persons with protan colour vision deficiency set the following values

- on the yellow slider y>0
- on the purple (purple) slider p>0
- on the turquoise (turquoise) slider t=0.

Persons with deuteran colour vision deficiency set the following values

- on the yellow slider y<0
- on the purple (purple) slider p=0
- on the turquoise (turquoise) slider t<0.

The numerical magnitude of the "+" and "-" values (0-50) show the severity of the given type of colour vision deficiency.

The type and severity of protan or deuteran colour blindness could be theoretically determined by the position of the yellow slider alone, but the redundancy resulting from the testing of various colours increases the reliability of the measurement.

Makin use of the calibration method in the case of the above colour vision test takes place in the following way.

If the calibration was performed with an A/4 paper sheet, then the $\Delta$=r-g value is available as display error (or as a component of it if, in addition to this, other factors are determined, such as brightness).

This value must be given to the colour vision measuring software as the initial step of the use of the colour vision test (for example the measuring software may ask for this and the patient has to type it in or, for example, the measuring software and the calibration software may communicate with each other, and the calibration software sends the $\Delta$ value to the measuring software straight away).

Taking account of the correction takes place in the following way.

If the correction is applied when displaying, then corrected colours are displayed within the circular surfaces 31a, 31b, 31c for the positions Y, P, T of the sliders 33a, 33b, 33c as follows:

In every position Y of the slider 33a the colour corresponding to the value y'=y+$\Delta$ is displayed in the circular surface 31a. If y'$\geq$0, then the intensity of the red subpixels 21 in the displayed colour is 255, and the intensity of the green subpixels 21 is 255-y', if y'<0, the intensity of the green subpixels 21 in the displayed colour is 255, and the intensity of the red subpixels 21 is 255+y'. The method has been illustrated schematically in Fig. 7a, where the colour corresponding to the value y' is displayed in the circular surface 31a. Here the values y>0 and $\Delta$>0 have been presumed, but naturally either one or both may be negative numbers, from the point of view of the displaying what is significant is whether the sum of the two is positive or negative. If the value y' is greater than +50 or smaller than -50 then in the case of displaying the intensity of the red and green subpixels 21 the rule applied within the scale is continued. If then the value of +50 is exceeded, in other words y'>50, then the intensity of the red subpixels 21 is still set to the value of 255, and the intensity of the green subpixels 21 is reduced to the value of 255-y'. If y'<(-50) then the intensity of the green subpixels 21 is maintained at the value of 255, while the intensity of the red subpixels 21 is set to the value of 255+y', where a negative number smaller than (-50) is added to the value 255, in other words the intensity of the red subpixels 21 will be smaller than 205. With this setting, if the person being tested also has normal colour vision, then with the slider 33a in its central position (y=0) even in spite of the display error he or she will see the colour yellow (neither reddish nor greenish shade of yellow) within the circular surface 31a.

If only those persons with the most common form of colour vision deficiency, i.e. protan and deuteran colour vision deficiency are to be tested, then it is sufficient to correct the displaying of the red and green colours on the sliders 33b and 33c (irrespective of any error in the colour blue).

Similarly to the above, the colour corresponding to the value p'=p+$\Delta$ is displayed within the circular surface 31b for every position P of the slider 33b according to the rules explained in the case of adjusting yellow. In other words with this setting if the person being tested is a person with normal colour vision, then with the slider 33b in its central position (p=0) even in spite of the display error he or she will see the colour purple in the circular surface 31b that has neither a bluish tone nor a reddish tone.

The colour corresponding to the value t'=t+$\Delta$ is displayed within the circular surface 31c for every position T of the slider 33c according to the rules explained in the case of adjusting yellow. With this setting if the person being tested is a person with normal colour vision then with the slider 33c in its central position (t=0) even in spite of the display error he or she will see the colour turquoise in the circular surface 31c that has neither a bluish tone nor a greenish tone.

Using the above method the y, p and t values set by the patient may serve as the basis of a diagnosis as the display error has already been corrected on the screen 12 when these values were inputted by the user.

Another possibility is that the displayed colours themselves are not changed during the measurement of colour vision, instead the y, p, t values corresponding to the read positions Y, P, T of the sliders 33a, 33b, 33c, respectively, as measurement results are subsequently modified according to the formulae y'=y-$\Delta$, p'=p-$\Delta$ and t'=t-$\Delta$, and it is these latter values that serve as the basis of the diagnosis. Here the display error $\Delta$ is subtracted from the y, p and t values corresponding to the positions of the sliders 33a, 33b, 33c, as the deviation from the 0 position is partially or completely caused by the display error and not by the colour blindness.

In connection with the calibration method it was mentioned earlier that an embodiment is conceivable in the case of which the r-b and b-g values are also determined as further components of the display error. In this case the formulae p'=p+(r-b) and t'=t+(b-g) may be used for the colours displayed at the P and T positions, or if the display error is not corrected in the case of displaying, then during diagnosis the calculations may be made with the formulae p'=p-(r-b) and t'=t-(b-g). In this way the very rare fault of the S-cone can be measured.

If calibration takes place with the person with normal colour vision performing the same test as the person with colour vision deficiency when performing the calibration, then the $y_2$, $p_2$, $t_2$ values belonging to every single setting made by the person with colour vision deficiency must be corrected with the same $y_1$, $p_1$, $t_1$ values

of the person with normal colour vision.

This may take place in two ways as well: In case the calibration software operates on the same principle as the measuring software but it is distinct therefrom, then the result y1, p1, t1 of the calibration software belonging to the positions Y1, P1, T1 set by the person with normal colour vision are transferred to the measuring software, and instead of displaying the colours corresponding to the values y2, p2 and t2 belonging to the positions Y2, P2 and T2 when the patient sets the colours, the colours corresponding to the values y2'=y2+y1, p2'=p2+y1 and t2'=t2+y1 are displayed within the circular surfaces 31a, 31b, 31c according to the rules described above. With this method the display error has been corrected in the same way as in the previous example by adding the value ∆=r-g, as the position Y1 of the slider 33a itself codes an r-g value increasing by increments of one between -50 and +50, since red and green intensities vary according to definite rules, but in such a way that a unit change of y1 always represents a unit change of r-g with the same sign. In this case the basis of the diagnosis are the y2, p2 and t2 values set and actually inputted by the patient.

It is also possible to take into account the intensity of the blue coloured subpixels 21 by using the values of p1 and t1 in the above formulae, in other words y2'=y2+y1, p2'=p2+p1 and t2'=t2+t1.

If, however, the calibration software results y1, p1, t1 of the person with normal colour vision are only recorded, but the calibration results are not used to modify the measuring software, then the final result must be subsequently corrected. If the focus is only on the display errors of the colours red and green, then the following formulae may be used:

$$y = y2 - y1$$

$$p = p2 - y1$$

$$t = t2 - y1$$

if, however, the colour blue is also to be taken into account, then the following formulae may be used:

$$y = y2 - y1$$

$$p = p2 - p1$$

$$t = t2 - t1$$

where

y2, p2, t2 are the values measured for the person with colour deficiency,

y1, p1, t1 are the settings made by the person with normal colour vision,

and the y, p, t values will be the amounts serving as the basis of the diagnosis.

In the above example it was shown how a task for testing colour vision may be provided by mixing two primary colours (subpixel 21 colours). In the present case the task for the user (patient) was to set a third colour formed from a mixture of two primary colours. The task may also involve colour selection instead of colour setting, here these are also jointly referred to as colour determination tasks. The colour determination task may also be performed with the use of more than two primary colours, the task, for example, may be the setting of various colour tones mixed from two or more primary colours, or their differentiation, or placing them in order, etc. In the case of the above example each colour setting task required the setting of one colour tone within the circular surface 31a, 31b 31c belonging to the given task, but other embodiments are conceivable in the case of which, similarly to the Ishihara test, shapes of various colour tones are displayed for each task, such as tiny circular coloured surfaces. It is obvious for a person skilled in the art that such more complex tasks may be derived from the examples presented above.

Various modifications to the above disclosed embodiments will be apparent to a person skilled in the art without departing from the scope of protection determined by the attached claims.

**Claims**

1. Method of calibrating a colour vision test for testing colour vision under given ambient lighting conditions, which colour vision test is to be displayed on a colour screen (12) of a computer (10) having at least one input interface (14), **characterised by** displaying a calibration test on the screen (12) under given ambient lighting conditions to a person with normal colour vision before starting the colour vision test,
displaying within at least one measuring region (31) of the screen (12) a colour determination task requiring user input as a part of the calibration test, reading the user input through at least one input interface (14) of the computer (10),
evaluating the read user input and determining as a result of the evaluation a display error resulting from a combination of a colour reproduction capability of the screen (12) and an effect of the ambient lighting conditions on colour vision, and
determining a modification to the colour vision test from the display error that corrects the colour vision test with respect to the display error.

**2.** Method according to claim 1, **characterised by** displaying a colour determination task that requires as user input the determination by the user of the intensity of at least a first coloured subpixels selected from red, green and blue subpixels (21) of pixels (20) of the measuring region (31) via a user input interface (14) of the computer (10),
reading the user input relating to the determination of the intensity of at least the first coloured subpixels (21) via the at least one input interface (14),
evaluating the read user input, and as a result of the evaluation determining the display error in respect of at least the first coloured subpixels (21), which display error is the deviation from a first reference colour corresponding to the at least first colour resulting from the combination of the colour reproduction capability of the screen (12) and of the effects of the ambient lighting conditions exerted on the vision of the first colour.

**3.** Method according to claim 2, **characterised by** displaying a colour determination task during which the colour indicated in the colour determination task has to be set or selected within the measuring range of the screen (12) by the user by setting or selecting the intensity of at least the first coloured subpixels (21) using the user input interface (14), and reading as user input the data relating to the intensity setting or selection of the at least first coloured subpixels (21) via the at least one input interface (14) are.

**4.** Method according to claim 2, **characterised by** displaying a colour determination task during which white or yellow colour has to be set within the measuring region (31) of the screen (12) by the user by setting the intensity of at least the red and green subpixels (21) using the user input interface (14), and during evaluation determining the difference in intensity of the red and green subpixels (21) within the measuring region (31) of the screen (12) from the read setting data, and regarding this as the display error.

**5.** Method according to claim 2, **characterised by** displaying a colour determination task during which purple (purple) or turquoise (turquoise) colours have to be set within the measuring region (31) of the screen (12) by the user by setting the intensity of the red and blue or the green and blue subpixels (21) using the user input interface (14), and during evaluation determining the difference in intensity of the red and blue or the green and blue subpixels (21) within the measuring region (31) of the screen (12) from the read setting data, and regarding this as the display error.

**6.** Method according to any one of claims 1 to 5, **characterised by** providing instructions for a person with normal colour vision during the calibration test before performing the colour determination task to look at an object (40) known to be white for a few minutes, preferably a white sheet of paper, preferably for at least 2 minutes, optimally for 10 minutes

**7.** Method according to claim 6, **characterised by** displaying a colour determination task during which white colour corresponding to the colour of the object (40) has to be set within the measuring region (31) of the screen (12) by the user by setting the intensity of at least the red and green subpixels (21) selected from the red, green and blue subpixels (21) of the pixels of the measuring region (31) using the user input interface (14), reading the setting data relating to the intensity of the red and green subpixels (21) through the at least one input interface (14) as user input, and during the evaluation determining the difference of the intensity of the green and red subpixels (21) within the measuring region (31) of the screen (12) from the read setting data, and regarding this as the display error.

**8.** Method according to any one of claims 1 to 7, **characterised by** checking whether the screen (12) has subpixels (21) of another colour apart from the red, green and blue subpixels (21), and if so setting its intensity to zero at least within the measuring region (31) during the displaying of the colour determination task.

**9.** Method of testing colour vision during which a colour vision test for testing colour vision is displayed on a screen (12) of a computer (10) having at least one input interface (14) under given ambient lighting conditions, **characterised by** performing the calibration method according to any one of claims 1 to 8 before starting the colour vision test
performing the modification to the colour vision test determined during the calibration method,
displaying the modified colour vision test on the screen (12),
displaying a colour determination task requiring user input within at least a measuring region (31, 31a, 31b, 31c) of the screen (12) as a part of the colour vision test,
reading the user input through the at least one input interface (14) of the computer,
evaluating the read user input, and determining as a result of the evaluation the colour vision of a user performing the colour vision test.

**10.** Method according to claim 9, **characterised by** determining a modification to the display parameters of the colour vision test as the modification to the colour vision test which corrects the colour vision test with respect to the display error when the colour vision test is displayed on the screen (12), and accordingly modifying the display parameters of the colour vision test when the colour vision test is modified, or determining a modification to the evaluation parameters of the colour vision test as the modification to the colour vision test which corrects the measurement result obtained with the colour vision test with respect to the display error when evaluating the colour vision test, and when modifying the colour vision test the evaluation parameters of the colour vision test are modified accordingly.

11. Method according to any of claims 9 or 10, **characterised by** the calibration test being the same as the colour vision test.

12. Computer (10) comprising a colour screen (12), at least one input interface (14), at least one processor (16) and a non-volatile storage medium (17) storing a computer program containing computer instructions, the computer program being configured such as to cause the computer to carry out any one of the methods according to claims 1 to 9 when said computer instructions are executed by the at least one processor (16).

13. Computer (10) according to claim 12, **characterised by** the computer program being configured such as to cause the computer (10) to carry out any one of the methods according to claims 10 to 12 after carrying out any one of the methods according to claims 1 to 9 when the computer instructions are executed by the at least one processor (16).

14. Computer program containing computer instructions, the computer program being configured such as to cause a computer (10) having at least one processor operatively connected to a colour screen (12) and at least one input interface (14) to carry out any one of the methods according to claims 1 to 9 when said computer instructions are executed by the at least one processor (16) of the computer (10).

15. Computer program according to claim 14, **characterised by** the computer program being configured such as to cause the computer (10) to carry out any one of the methods according to claims 10 to 12 after carrying out any one of the methods according to claims 1 to 9 when the computer instructions are executed by the at least one processor (16).

Fig. 1

Fig. 2a

Fig. 2b

Fig. 2c

S10 — Displaying calibration test

↓

S101 — Instructions to adapt eye

↓

S102 — Colour determination exercise

↓

S11 — Reading user input

↓

S12 — Evaluating user input

↓

S13 — Display error

↓

S14 — Determining modification of colour vision test

Fig. 3

S15 — Calibration test (for patient)

S151 — Instructions to adapt eye

S152 — Colour determination exercise

S16 — Reading value g

S17 — Modifying colour vision test with value g

Fig. 3a

EP 3 838 112 A1

set background colour to white using
the colour sliders and the brightness
slider

32

R          ⇩

G          ⇩

B          ⇩

33

31

12

☀          ⇩

34

40

Fig. 4

S19 — Modifying colour vision test

S20 — Displaying colour vision test

S201 — Instructions to adapt eye

S202 — Colour determination exercise

S21 — Reading user input

S22 — Evaluating user input

S23 — Determining colour vision

Fig. 5

EP 3 838 112 A1

set background colour to yellow

32

R

G

31'

33

12

40

34

Fig. 6

Fig. 7

slider:

r:  205                    255      255              255

g:  255                    255      255-y            205

y:  -50                    0        y                +50

display:

+Δ

r:  205                    255      255              255

g:  255                    255      255-(y+Δ)        205

r-g:  -50                  0        y→(y')=y+Δ       +50

31a

Fig. 7a

Europäisches
Patentamt

European
Patent Office

Office européen
des brevets

# EUROPEAN SEARCH REPORT

Application Number

EP 19 21 7946

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 946 075 A (HORN GERALD [US]) 31 August 1999 (1999-08-31) * the whole document * | 1-15 | INV. A61B3/00 |
| A | US 2010/188639 A1 (CARDA DAN D [US] ET AL) 29 July 2010 (2010-07-29) * paragraph [0011] * * paragraph [0025] - paragraph [0061] * | 1-15 | |
| A | WO 96/39919 A1 (REUTERS HEALTH INFORMATION SER [US]) 19 December 1996 (1996-12-19) * page 4, line 24 - page 5, line 6 * | 1-15 | |
| A | US 2013/293846 A1 (NORDSTROM CHERYL [US] ET AL) 7 November 2013 (2013-11-07) * paragraph [0026] * | 1-15 | |

TECHNICAL FIELDS
SEARCHED (IPC)

A61B

The present search report has been drawn up for all claims

| Place of search | Date of completion of the search | Examiner |
|---|---|---|
| Munich | 25 May 2020 | Alvazzi Delfrate, S |

EPO FORM 1503 03.82 (P04C01)

## ANNEX TO THE EUROPEAN SEARCH REPORT
## ON EUROPEAN PATENT APPLICATION NO.

EP 19 21 7946

This annex lists the patent family members relating to the patent documents cited in the above-mentioned European search report.
The members are as contained in the European Patent Office EDP file on
The European Patent Office is in no way liable for these particulars which are merely given for the purpose of information.

25-05-2020

| Patent document cited in search report | | Publication date | Patent family member(s) | | Publication date |
|---|---|---|---|---|---|
| US 5946075 | A | 31-08-1999 | US 5946075 A | | 31-08-1999 |
| | | | US 6260970 B1 | | 17-07-2001 |
| US 2010188639 | A1 | 29-07-2010 | US 2010188639 A1 | | 29-07-2010 |
| | | | WO 2010085726 A1 | | 29-07-2010 |
| WO 9639919 | A1 | 19-12-1996 | AU 712602 B2 | | 11-11-1999 |
| | | | CA 2231455 A1 | | 19-12-1996 |
| | | | EP 0833583 A1 | | 08-04-1998 |
| | | | NZ 310141 A | | 25-11-1998 |
| | | | US 5589898 A | | 31-12-1996 |
| | | | WO 9639919 A1 | | 19-12-1996 |
| US 2013293846 | A1 | 07-11-2013 | US 9883794 B1 | | 06-02-2018 |
| | | | US 2013293846 A1 | | 07-11-2013 |
| | | | US 2014218692 A1 | | 07-08-2014 |
| | | | US 2015036104 A1 | | 05-02-2015 |
| | | | WO 2013166473 A1 | | 07-11-2013 |

EPO FORM P0459

For more details about this annex : see Official Journal of the European Patent Office, No. 12/82